(19) Europäisches Patentamt
European Patent Office
Office européen des brevets

(11) **EP 0 581 624 B1**

(12) **FASCICULE DE BREVET EUROPEEN**

(45) Date de publication et mention de la délivrance du brevet:
**07.01.1999 Bulletin 1999/01**

(51) Int. Cl.$^6$: **A61K 7/48**, C11B 3/12, A23L 1/30, A23L 1/302, C11B 5/00

(21) Numéro de dépôt: **93401650.2**

(22) Date de dépôt: **25.06.1993**

(54) **Compositions cosmétique et/ou alimentaire contenant une fraction insaponifiable d'huile de sesame et de la vitamine E**

Kosmetisches und/oder Nahrungsmittel enthaltend eine nicht-verseifbare Fraktion von Sesamöl und Vitamin-E

Cosmetic or food composition containing a fraction of unsaponifiabler of hot oil and a vitamin-E

(84) Etats contractants désignés:
**AT BE CH DE DK ES FR GB GR IE IT LI LU MC NL PT SE**

(30) Priorité: **25.06.1992 FR 9207830**

(43) Date de publication de la demande:
**02.02.1994 Bulletin 1994/05**

(73) Titulaire: **EXPANCHIMIE S.A.R.L.**
**F-92400 Courbevoie (FR)**

(72) Inventeurs:
- **Rancurel, Alain**
  **F-28300 Mainvilliers (FR)**
- **Laigneau, François**
  **F-78150 Le Chesnay (FR)**

(74) Mandataire:
**Grosset-Fournier, Chantal Catherine**
**Grosset-Fournier & Demachy s.a.r.l.**
**103 rue La Fayette**
**75010 Paris (FR)**

(56) Documents cités:

| | |
|---|---|
| WO-A-85/00746 | DE-A- 2 019 226 |
| FR-A- 2 102 888 | FR-A- 2 648 347 |
| FR-A- 2 653 974 | FR-A- 2 656 219 |
| US-A- 2 126 466 | US-A- 2 396 679 |
| US-A- 4 454 159 | |

- PATENT ABSTRACTS OF JAPAN vol. 12, no. 421 (C-541)8 Novembre 1988
- S.T.N. File Supplier, KARLSRUHE, DE, File Chemical Abstracts, vol 116, n 5392
- SEIFEN-OLE-FETTE-WACHSE vol. 113, no. 16, 1987, DE pages 589 - 591 SANDHOFER 'der Weizenkeim-Quelle fur naturlische Kosmetik-Wirkstoffe'

**Description**

La présente invention a pour objet de nouvelles compositions à base de fractions insaponifiables d'huiles de germe de blé et de sésame, ainsi que leurs utilisations, notamment en cosmétologie et en qualité de complément alimentaire.

Des compositions contenant de l'huile de sésame en mélange avec du tocophérol et du sorbitol, utilisées pour la conservation de denrées périssables, sont décrites dans la demande japonaise JP-A-63152965.

Par ailleurs, des compositions comprenant principalement une lécithine, de la vitamine E, de l'huile de germe de blé et un mélange d'huiles végétales (comprenant notamment de l'huile de sésame), utilisées dans le cadre des soins de la peau liés au rasage, sont décrites dans la demande internationale WO-A-8500746.

Des préparations dermatologiques contenant du trioléate de glycérol, des huiles végétales (dont notamment l'huile de sésame ou l'huile de germe de blé), une huile hydrogénée, du tocophérol, de la lécithine et des acides gras, utilisées principalement dans le cadre du traitement des peaux sèches et irritées et du prurit, sont décrites dans le brevet US-A-4454159.

Selon la Pharmacopée européenne 2° édition page V.3.4.7., le terme "insaponifiable" s'applique aux substances, non volatiles, obtenues par extraction, avec un solvant organique, d'une solution de la substance à examiner après saponification d'une huile végétale ou animale.

La méthode de distillation moléculaire, qui sera davantage détaillée dans la suite de ce texte, permet de concentrer des huiles, issues notamment de plantes (huiles végétales), et d'obtenir des concentrés, encore désignés ci-après par concentrats, dans lesquels la concentration en insaponifiable peut atteindre de l'ordre de 10 à 20%, cette concentration étant de l'ordre de 1 à 2% dans les huiles de départ.

Des études réalisées par les inventeurs à partir de concentrats d'huile de germe de blé et d'huile de sésame, tels que ceux obtenus par distillation moléculaire de ces huiles, ont abouti aux résultats suivants:

- les tests réalisés par une technique de bioluminescence (dans laquelle les radicaux superoxydes produits par le système hypoxanthine/xanthine oxydase génèrent une émission de photons), montrent que le concentrat d'huile de germe de blé exerce un effet piégeur des radicaux libres oxygénés (en particulier des ions superoxydes $O_2^-$), légèrement inférieur à celui de la vitamine E,
- l'effet piégeur de radicaux hydroxyles a été testé par la méthode de dosage par colorimétrie du malondialdehyde produit par la dégradation du désoxyribose par les radicaux libres générés par le système hypoxanthine/xanthine oxydase; l'activité du concentrat d'huile de germe de blé apparaît peu significative en comparaison à celle du concentrat d'huile de sésame, cette dernière étant toutefois inférieure à celle mesurée dans les mêmes conditions expérimentales pour la vitamine E.

C'est dans le but d'obtenir une composition présentant à la fois un effet piégeur de radicaux hydroxyles comparable à celui du concentrat d'huile de sésame, ainsi qu'un effet piègeur des radicaux libres oxygénés et une activité cytoprotectrice comparables à ceux du concentrat d'huile de germe de blé, que les inventeurs ont préparé une composition constituée d'un mélange des deux concentrats susmentionnés.

L'action antiradicalaire de la composition ainsi obtenue a été mesurée dans des conditions opératoires définies ci-après, et comparée à cette même action mesurée pour le concentrat d'huile de germe de blé, d'huile de sésame et pour la vitamine E.

Les inventeurs ont ainsi déterminé que, de façon inattendue, la composition susmentionnée constituée du mélange des deux concentrats, présente la caractéristique de posséder une activité antiradicalaire vis à vis de l'irradiation aux UVA qui est d'une part supérieure à celle présentée par le concentrat d'huile de germe de blé, et, d'autre part supérieure à celle présentée par le concentrat d'huile de sésame.

L'activité antiradicalaire de la composition susmentionnée est également supérieure à celle mesurée pour la vitamine E dont la propre activité est elle-même supérieure à celle de chacun des deux concentrats susmentionnés.

Les inventeurs ont également pu constater que, de façon toute autant inattendue, la composition susmentionnée constituée du mélange de ces deux concentrats, est active non seulement à titre préventif ou immédiat contre les effets des UVA, mais également dans le cadre du traitement curatif des effets des UVA, alors que la vitamine E et les deux concentrats susmentionnés ne présentent pratiquement pas, voire aucune, action curative contre ces effets.

Ainsi la présente invention concerne, d'une manière générale, toute composition comprenant la fraction insaponifiable d'huile de sésame en mélange avec de la vitamine E, la proportion d'insaponifiable d'huile de sésame dans les compositions de l'invention étant de 10% à 20% en poids.

De préférence, la proportion de vitamine E dans la composition susmentionnée, est comprise entre environ 0,5% et environ 5% en poids, et avantageusement entre environ 1% et environ 3%.

L'invention a plus particulièrement pour objet toute composition telle que décrite ci-dessus comprenant la fraction insaponifiable d'huile de sésame en mélange avec une (ou plusieurs) fraction(s) insaponifiable(s) provenant de toute huile, notamment d'une huile végétale, susceptible de contenir de la vitamine E, notamment d'huile de soja, et avantageusement d'huile de germe de blé.

La présente invention vise plus particulièrement toute composition telle que décrite ci-dessus comprenant la fraction insaponifiable d'huile de germe de blé en mélange avec la fraction insaponifiable d'huile de sésame.

L'invention a plus particulièrement pour objet toute composition telle que décrite ci-dessus, et caractérisée en ce que les fractions insaponifiables d'huile de germe de blé et d'huile de sésame sont celles contenues dans les concentrats respectifs de ces huiles, ces concentrats étant avantageusement préparés par distillation moléculaire de ces huiles.

La méthode de distillation moléculaire susmentionnée est réalisée par étalement de l'huile en couche mince sur la surface chauffée d'un rotor conique tournant à grande vitesse. On maintient un vide poussé de l'ordre de $10^{-3}$ mm Hg (soit environ 0,13 Pa) dans l'enceinte de distillation.

Dans ces conditions il y a évaporation, et non pas ébullition, depuis la surface chaude, des constituants de l'insaponifiable dont la séparation devient possible par rapport aux glycérides, l'avantage étant que l'huile et l'insaponifiable, - ces produits réputés fragiles -, ne sont pas dégradés au cours de l'opération.

Les températures de distillation sont avantageusement comprises entre environ 190°C et environ 260°C, avantageusement entre environ 200°C et environ 220°C, et sous vide de l'ordre d'environ 0,1 Pa à environ 1,5 Pa, avantageusement d'environ 0,13 Pa à environ 0,5 Pa.

De préférence les quantités en poids des fractions insaponifiables dans les concentrats d'huile de germe de blé et d'huile de sésame sont respectivement d'environ 15% à environ 25%, et d'environ 10% à environ 20%.

Avantageusement, dans le mélange, les quantités en poids des concentrats d'huile de germe de blé et d'huile de sésame varient respectivement entre environ 10% à environ 90%, et entre environ 90% à environ 10%, de manière à ce que le total des quantités de ces deux huiles fasse 100%.

De préférence les quantités en poids des concentrats d'huile de germe de blé et d'huile de sésame sont respectivement de l'ordre de 50%.

D'une manière générale, les compositions préférées de l'invention comprennent environ 10 à 20% en poids de fractions insaponifiables, environ 10% en poids d'acides gras, le reste étant constitué de mono, di et triglycérides.

L'invention a également pour objet toute composition cosmétique comprenant une composition telle que décrite ci-dessus selon l'invention, de préférence en association avec un véhicule physiologiquement acceptable.

Les compositions cosmétiques selon l'invention sont avantageusement formulées pour l'usage topique, notamment sous forme de crèmes, d'émulsions, de pommades, ou encore sous une forme appropriée pour l'application labiale (en vue notamment du conditionnement dans des sticks labiaux).

Les compositions selon l'invention peuvent également être administrées par voie orale, notamment sous forme de gélules ou comprimés.

L'invention vise également l'utilisation des compositions susmentionnées pour la prévention et/ou le traitement de la peau d'un individu, contre les effets des UVA.

A ce titre les compositions susmentionnées sont plus particulièrement utilisées en tant que produits solaires traitants, crèmes restructurantes, nutritives, antirides (lutte contre le vieillissement de la peau), et protectrices de jour.

L'invention a également pour objet toute méthode de traitement esthétique comprenant l'application sur la surface cutanée d'un individu d'une composition selon l'invention.

L'invention vise également l'utilisation d'une composition telle que décrite ci-dessus en tant que composition alimentaire (notamment en tant que complément ou supplément nutritionnel).

L'invention concerne également toute composition pharmaceutique comprenant une composition telle que décrite ci-dessus en association avec un véhicule physiologiquement acceptable.

Les compositions pharmaceutiques selon l'invention sont plus particulièrement destinées à la prévention ou au traitement des effets des UVA sur la peau, et à la prévention du vieillissement de la peau.

L'invention a également pour objet un procédé de fabrication des compositions décrites ci-dessus, ce procédé comprenant:

- soit le mélange des deux concentrats d'huile de germe de blé et d'huile de sésame tels qu'obtenus par la méthode de distillation moléculaire décrite dans la revue "Parfumerie cosmétique et arômes" (1985), n° 61, pages 91-96,

- soit le mélange préalable des huiles de germe de blé et de sésame, suivi de la distillation moléculaire selon le procédé décrit ci-dessus.

Les concentrats susmentionnés sont avantageusement obtenus par distillation moléculaire selon le procédé décrit ci-dessus. Ils peuvent cependant être obtenus par d'autres méthodes, notamment par cristallisation par le froid (voir par exemple l'article de J. JACINI: J. ASS. OFF. AN. CHEM., 1967, 50, p.85-90), ou encore par extraction liquide-liquide (voir par exemple l'article de H. KALLEL: REV. FRANC. DES CORPS GRAS, 1975, 22, 5, p.269-270).

L'invention sera davantage détaillée dans ce qui suit à l'aide d'exemples de fabrication des concentrats d'huile de germe de blé et d'huile de sésame et de leur mélange selon l'invention, de formulations de compositions à base de ce mélange, ainsi que des mesures des effets anti-UVA de ce dernier.

## I - Exemples de préparation des deux concentrats et de leurs mélanges Exemple 1 - L'huile de sésame

On introduit dans un appareil de distillation moléculaire approprié, et de préférence du type centrifuge, de l'huile de sésame: brute, vierge, semi-raffinée ou raffinée avec un débit d'alimentation de 10 à 30 kgs par heure et de préférence entre 15 et 20.

Les paramètres de la distillation sont les suivants:

- température: 190 à 220°C,
- vide de 1 à 10µ (soit 0,13 à 1,3 Pa).

Le pourcentage distillé étant proche de 10, on recueille soigneusement ce distillat.
La richesse en matière non saponifiable de cette fraction distillée est comprise entre 10% et 20%.

## Exemple 2 - L'huile de germe de blé

On introduit dans un appareil de distillation moléculaire approprié tel que décrit ci-dessus, de l'huile de germe de blé brute, vierge, semi-raffinée avec un débit d'alimentation de 10 à 30 kgs par heure et de préférence entre 15 et 25.

Les paramètres de la distillation sont les suivants:

- température: 200 à 250°C,
- vide de 1 à 10µ (soit 0,13 à 1,3 Pa).

Le pourcentage distillé étant compris entre 6 et 12, on recueille soigneusement ce distillat.
La richesse en matière non saponifiable de cette fraction distillée est comprise entre 15% et 30%.

## Exemple 3 - Préparation du mélange des deux concentrats des exemples 1 et 2

La préparation proprement dite du mélange peut être effectuée dans un mélangeur classique muni d'une ancre, l'opération se faisant sous atmosphère inerte en chauffant à une température voisine de 60°C.

## II - Exemples de formules 1.Crème nutritive - émulsion E/H

|  | % |
| --- | --- |
| Polyisobutène hydrogéné | 4,00 |
| Triglycérides caprylique caprique | 3,00 |
| Huile minérale et alcools de lanoline | 2,50 |
| Myristate de myristyle | 2,00 |
| Phosphate trioléate | 2,00 |
| Alcool oléique 2 OE | 2,00 |
| Palmitate de cétyle | 1,50 |
| Copolymère peg 45 dodécyl glycol | 1,50 |
| **Concentrats d'huile de sésame et d'huile de germe de blé** | 1,00 |
| Triglycérides C18-36 | 1,00 |
| Sulfate de magnésium | 0,30 |
| Conservateur GD 700 | 0,30 |
| Parfum | 0,30 |

(suite)

|  | % |
|---|---|
| Eau purifiée       Q.s.p. | 100,00 |

## 2 - Crème restructurante - émulsion H/E

|  | % |
|---|---|
| Cétéaryl glucoside | 4,00 |
| Myristate de myristyle | 3,00 |
| **Concentrats d'huile de sésame et d'huile de germe de blé** | 2,00 |
| Polysilane 70641 V 200 | 2,00 |
| Octyl dodécanol (Eutanol G) | 2,00 |
| Squalane (Perhydrosqualène naturel) | 2,00 |
| Huile de jojoba | 2,00 |
| Stéarate de diéthylène glycol | 2,00 |
| Alcool cétostéarylique 25 OE | 1,00 |
| Parfum | 0,30 |
| Conservateur GD 700 | 0,20 |
| Citrate trisodique | 0,10 |
| Eau purifiée       q.s.p. | 100,00 |

## 3. Huile solaire indice de protection 6

|  | % |
|---|---|
| Dipélargonate de propylène glycol | 61,00 |
| Cyclométhicone (Dow Corning 344 fluid) | 10,00 |
| Citrate de trioctyle | 10,00 |
| Maléate de dioctyle | 10,00 |
| Méthoxycinnamate d'octyle | 4,00 |
| **Concentrats d'huile de sésame et d'huile de germe de blé** | 2,00 |
| Benzophénone 3 | 2,00 |
| Parfum | 1,00 |

## III - NORMES DES CONCENTRATS

## 1 - CONCENTRAT D'HUILE DE SESAME

- Caractères organoleptiques: liquide huileux de couleur jaune orangé, d'odeur caractéristique, présentant un dépôt cristallisé blanc.

- Solubilités:

| | |
|---|---|
| éther éthylique | soluble |
| chloroforme | soluble |
| éthanol | insoluble |

- Indice de peroxyde: ≤10 milliéquivalents/kg
- Indice d'acide: ≤20
- Absorbance spécifique à 287 nm: ≥15
- Composition en acides gras :

| | |
|---|---|
| Acide palmitique $C_{16}$ | 5 à 20 % |
| Acide palmitoléique $C_{16'}$ | 0,1 à 1 % |
| Acide stéarique $C_{18}$ | 4 à 8 % |
| Acide oléique $C_{18'}$ | 35 à 45 % |
| Acide linoléique $C_{18''}$ | 30 à 47 % |
| Acide linolénique $C_{18'''}$ | 0,1 à 1 % |
| Acide arachidique $C_{20}$ | 0,1 à 1,5% |
| - Teneur en insaponifiable | 10 à 20 g/100 g |
| - Teneur en stérols | 3 à 8 g/100 g |
| - Teneur en sésamine | ≥4 g/100 g |
| - Teneur en sésamoline | ≥2 g/100 g |

**2- CONCENTRAT D'HUILE DE GERME DE BLE**

- Caractères organoleptiques: liquide huileux translucide, de couleur jaune orangé, d'odeur caractéristique.
- Solubilités:

| | |
|---|---|
| Hexane | Soluble |
| Chloroforme | Soluble |
| Alcool | Partiellement soluble |

- Indice d'acide: <20
- Indice de peroxyde: ≤10 milliéquivalents/kg
- Teneur en insaponifiable: 15 à 25%
- Teneur en tocophérols: >1%
- Teneur en stérols totaux: 5 à 20%

  . % relatif en campestérols: 15 à 30%
  . % relatif en stigmastérol: < 2 %
  . % relatif en β-sitostérol: 50 à 70 %

Les teneurs en insaponifiable sont mesurée par la méthode de la pharmacopée Européenne 2° édition page V.3.4.7.

**IV - ETUDE DE L'ACTIVITE ANTIRADICALAIRE DE CONCENTRATS D'HUILE DE SESAME, DE GERME DE BLE, ET DE LEUR MELANGE: COMPARAISON AVEC LA VITAMINE E.**

**1. PRODUITS ESSAYES**

Trois échantillons de concentrats référencés ci-après ont été essayés:
CSS lot 123061: concentrat de sésame (environ 13% d'insaponifiable).
CGB lot 102100: concentrat de germe de blé (environ 20% d'insaponifiable).
CSB lot 137111: mélange des deux (50% du concentrat d'huile de sésame et 50% de concentrat d'huile de germe de blé).
L'activité de ces trois lots a été comparée à celle d'une solution de vitamine E ($\alpha$ tocophérol Merck dosé à 92% de pureté par HPLC).

**2. MATERIEL ET METHODES**

**2.1. Cultures cellulaires.**

Les cellules sont des fibroblastes isolés à partir d'un prépuce d'un jeune enfant.

**2.2. Evaluation de la viabilité cellulaire.**

La viabilité cellulaire a été quantifiée par une méthode colorimètrique basée sur le métabolisme mitochondrial par les cellules d'un sel de tétrazolium (le MTT).

**2.3. Irradiation par les UVA.**

**2.3.1. Génération de radicaux libres.**

Les radicaux libres sont générés au niveau de la culture cellulaire par irradiation par les rayonnements ultraviolets A. La lampe utilisée est une lampe Vilber Lourmat (T40M) à vapeur de mercure dont le spectre d'émission est maximal à 365 nm. L'intensité d'irradiation est contrôlée par un radiomètre. Une intensité d'irradiation de 2,3 Joules/cm$^2$ permet d'obtenir une toxicité sur les fibroblastes voisine de 70% 24 heures après l'irradiation. Les cellules sont exposées, après 48 heures de culture, aux rayonnements à travers une solution de Hanks sans rouge de phénol. Après l'exposition, la solution saline est éliminée et les cellules sont remises en contact avec leur milieu de culture pendant 24 heures. La viabilité cellulaire est ensuite déterminée par le test au MTT. Le schéma suivant montre le protocole suivi:

$$J_0\text{-------------------------}J_2\text{---------------------------}J_3\text{----------------------------}$$

milieu             milieu            viabilité

^                      MTT

^

^

irradiation UVA

2,3 j/cm²

solution de Hanks

### 2.3.2. Protocole d'administration des produits.

Les propriétés antiradicalaires de chaque produit ont été recherchées après administration préventive, lors d'une administration simultanée (immédiat) à l'agression ou par administration après irradiation (curatif).

Enfin les protocoles précédents ont été associés.

1) administration préventive.

0,1 ml de chaque dilution du produit dans le milieu de culture complet sont introduits dans les puits, puis l'ensemencement est réalisé avec 0,1 ml de suspension cellulaire (200 000 cellules/ ml). Le temps de contact avec la culture cellulaire est de 48 heures ($J_0$ - $J_2$).

2) administration immédiate.

Dans ce cas, les actifs sont dans une solution de Hanks sans rouge de phénol aux différentes concentrations précédentes. Après irradiation des cellules, la solution de Hanks est éliminée et remplacée par du milieu complet pendant 24 heures puis la survie cellulaire est déterminée.

3) administration curative.

Les dilutions du produit dans le milieu de culture complet sont ajoutées immédiatement après l'irradiation et le temps de contact avec la culture est de 24 heures.

4) protocoles associés.

Les différents protocoles peuvent être associés entre eux selon le mode suivant:

Préventif + immédiat.
Immédiat + curatif.
Préventif + immédiat + curatif.

### 2.3.3. témoins.

Différents témoins non irradiés sont réalisés:

- Témoin absolu avec du MEM complet,
- Témoins solvants renfermant respectivement 1% d'éthanol ou 0,5% de diméthylformamide,
- Témoins actifs non irradiés à chaque concentration d'actif étudiée.

## 3. RESULTATS

### 3.1. Cytotoxicité (tableau I)

Le diméthylformamide à 1% présente une cytotoxicité non négligeable qui entraîne la mort d'environ 1/3 des cellules.

A la concentration de 0,1 g/l, une cytotoxicité apparaît pour les 3 concentrats; celle-ci est due au diméthylformamide qui est à la concentration de 1% dans ces conditions.

Pour les concentrations en extraits plus faibles, réalisées par dilutions successives, il n'y a pas de cytotoxicité apparente.

A la concentration de 0,05 g/l en concentrat, le pourcentage de diméthylformamide est de 0,5% qui n'est pas cytotoxique. C'est cette concentration qui sera utilisée dans les essais d'irradiation.

### 3.2. Action antiradicalaire vis à vis des UVA.

### 3.2.1. Vitamine E (tableau II)

En préventif, la vitamine E protège totalement les cellules contre les UVA jusqu'à la concentration de 10 µg/ml.

En immédiat, un effet protecteur moins puissant est également observé. Un effet filtre lié au trouble des solutions peut se surajouter aux plus fortes concentrations.

La vitamine E ne présente pratiquement pas d'effet curatif selon les différentes séries d'expériences présentées dans les tableaux II et VI.

L'association des traitements confirme les effets de la vitamine E en préventif et en traitement immédiat.

### 3.2.2. Concentrat de sésame (tableau III)

Le concentrat de sésame exerce un effet préventif jusqu'à la concentration de 10 $\mu$g/ml. Il ne présente pas d'effet immédiat (un léger effet est observé aux plus fortes concentrations; celui ci est lié à un effet filtre produit par le trouble du milieu), ni d'effet curatif.

Lorsque les traitements immédiat + curatif ou préventif + immédiat + curatif sont associés, l'action est supérieure à l'additivité des effets des traitements séparés. Pour les 3 traitements associés, une protection de 86% est obtenue à 5 $\mu$g/ml tandis qu'à cette concentration les traitements séparés sont sans effets.

### 3.2.3. Concentrat de germe de blé (tableau IV)

Le concentrat de germe de blé possède un effet préventif jusqu'à 5 $\mu$g/ml (dose la plus faible étudiée dans ce cas). Il ne présente aucun effet en immédiat et en curatif quelque soit la concentration utilisée.

L'association des 3 protocoles permet une protection à 100% à toutes les concentrations d'actif étudiées.

### 3.2.4. Mélange des 2 concentrats (tableau V)

Le mélange des 2 concentrats permet d'obtenir un effet protecteur très important de l'ordre de 90% vis à vis des UVA jusqu'à la concentration de 5 $\mu$g/ml. Le mélange n'est pas actif en immédiat.

Le traitement curatif révèle une efficacité importante d'environ 90% de ce mélange aux 2 plus fortes concentrations (100 et 50 $\mu$g/ml).

En associant traitement immédiat et curatif, l'efficacité est de l'ordre de 60% jusqu'à la concentration de 5 $\mu$g/ml confirmant ainsi l'effet curatif observé.

L'association des 3 protocoles permet d'obtenir une protection de 90% jusqu'à 5 $\mu$g/ml.

### 3.2.5. Mélange des 2 concentrats en proportions variables(tableau VI)

Le tableau VI représente l'efficacité en pourcentage de la vitamine E, du concentrat de blé pur, du concentrat de sésame pur, et des mélanges de ces deux derniers concentrats en proportions variables (33%/66%, 50%/50%, et 66%/33% respectivement), sur culture de fibroblastes irradiés, en traitement curatif.

Une forte sensibilité aux U.V. de la souche de cellules a permis de préciser les résultats précédents; on constate ainsi que la vitamine E seule est dénuée d'activité, ainsi que le concentrat de sésame.

A l'inverse, dès que l'on réalise un mélange, on voit la synergie se manifester avec un maximum pour le mélange 50/50, à toutes les concentrations étudiées.

### 4. CONCLUSION

Les 3 concentrats présentent tous une activité antiradicalaire vis à vis de l'irradiation aux UVA.

Ils se différencient par la puissance de leur action et le moment où leur activité apparait (préventif, immédiat, curatif).

En traitement préventif, les 3 concentrats sont efficaces; ils peuvent être classés par ordre d'activité décroissante:

mélange ≥germe de blé> sésame

Les différents concentrats ne présentent pas d'action en immédiat.

Enfin, le mélange germe de blé-sésame révèle une activité en traitement curatif jusqu'à la concentration de 10 $\mu$g/ml. Il est important de remarquer que ces effets n'apparaissent pas avec les concentrats de germe de blé ou de sésame administrés séparément ni avec la vitamine E.

Aussi, le mélange des 2 concentrats fait apparaître une action curative vis à vis de la toxicité induite par les UVA qui n'existe pas pour la plupart des antiradicalaires classiquement utilisés.

Tableau I: Cytotoxicité en % par rapport aux témoins des concentrats sur culture de fibroblastes après 48 heures

| produit | témoins absolus | témoins DMF 1% | Concentration | | | | |
|---|---|---|---|---|---|---|---|
| | | | 100 $\mu$g/ml | 50 $\mu$g/ml | 10 $\mu$g/ml | 5 $\mu$g/ml | 1 $\mu$g/ml |
| concentrat de sésame | 0 | 33 | 40 | 7 | 0 | 4 | 0 |
| concentrat de germe de blé | 0 | 36 | 35 | 10 | 7 | 12 | 7 |
| mélange des 2 | 0 | 36 | 30 | 3 | 0 | 1 | 1 |

| Tableau II: EFFICACITE EN % DE LA VITAMINE E |
|---|

| modalités de traitement | Concentration | | | | |
|---|---|---|---|---|---|
| | 1000 $\mu$g/ml | 500 $\mu$g/ml | 100 $\mu$g/ml | 50 $\mu$g/ml | 10 $\mu$g/ml |
| préventif (p) | 100 | 100 | 100 | 100 | 100 |
| immédiat (i) | 100 | 95 | 88 | 81 | 69 |
| p + i | 100 | 100 | 100 | 100 | 100 |
| curatif (c) | 26 | 22 | 38 | 29 | 22 |
| i + c | 77 | 72 | 85 | 91 | 72 |
| p + i + c | 100 | 100 | 100 | 100 | 100 |

| Tableau III: EFFICACITE EN % DU CONCENTRAT DE SESAME |
|---|

| modalités de traitement | Concentration | | | | |
|---|---|---|---|---|---|
| | 100 $\mu$g/ml | 50 $\mu$g/ml | 100 $\mu$g/ml | 5 $\mu$g/ml | 1 $\mu$g/ml |
| préventif | 91 | 100 | 34 | 0 | 0 |
| immédiat | 37 | 16 | 0 | 0 | 0 |
| p + i | 83 | 87 | 55 | 0 | 0 |
| curatif | 0 | 0 | 0 | 0 | 0 |
| i + c | 81 | 47 | 25 | 16 | 0 |
| p + i + c | 100 | 98 | 100 | 86 | 38 |

Tableau IV: EFFICACITE EN % DU CONCENTRAT DE GERME DE BLE

| modalités de traitement | concentration | | | |
|---|---|---|---|---|
| | 100 $\mu$g/ml | 50 $\mu$g/ml | 10 $\mu$g/ml | 5 $\mu$g/ml |
| *préventif* | 88 | 90 | 75 | 63 |
| *immédiat* | 0 | 0 | 0 | 0 |
| *p + i* | 76 | 94 | 85 | 84 |
| *curatif* | 20 | 14 | 14 | 10 |
| *i + c* | 34 | 37 | 22 | 22 |
| *p + i + c* | 100 | 100 | 100 | 98 |

Tableau V: EFFICACITE EN % DU MELANGE DES 2 CONCENTRATS

| modalités de traitement | Concentration | | | | |
|---|---|---|---|---|---|
| | 100 $\mu$g/ml | 50 $\mu$g/ml | 10 $\mu$g/ml | 5 $\mu$g/ml | 1 $\mu$g/ml |
| *préventif* | 97 | 90 | 96 | 81 | 2 |
| *immédiat* | 18 | 14 | 8 | 0 | 0 |
| *p + i* | 100 | 90 | 90 | 87 | 0 |
| *curatif* | 82 | 100 | 39 | 6 | 0 |
| *i + c* | 57 | 73 | 58 | 21 | 2 |
| *p + i + c* | 95 | 92 | 80 | 65 | 21 |

Tableau VI: EFFICACITE EN % DE LA VITAMINE E, DU CONCENTRAT DE BLE PUR, DU CONCENTRAT DE SESAME PUR, DU MELANGE DES CONCENTRATS DE SESAME ET DE BLE EN PROPORTIONS VARIABLES, SUR CULTURE DE FIBROBLASTES IRRADIES, EN TRAITEMENT CURATIF

| Produits et leurs mélanges | Concentration | | | | |
|---|---|---|---|---|---|
| | 100 $\mu$g/ml | 50 $\mu$g/ml | 10 $\mu$g/ml | 5 $\mu$g/ml | 1 $\mu$g/ml |
| *blé pur* | 13 | 14 | 0 | 0 | 0 |
| *sésame pur* | 0 | 0 | 0 | 0 | 0 |
| *blé/sésame 33%/66%* | 19 | 13 | 1 | 0 | 0 |
| *blé/sésame 50%/50%* | 34 | 44 | 14 | 5 | 0 |
| *blé/sésame 66%/33%* | 21 | 12 | 4 | 4 | 0 |
| | Concentration $\mu$g/ml | | | | |
| | 1000 | 500 | 100 | 50 | 10 |
| *vitamine E* | 0 | 0 | 0 | 0 | 0 |

## Revendications

1. Composition comprenant la fraction insaponifiable d'huile de sésame en mélange avec de la vitamine E, la proportion de cette fraction insaponifiable d'huile de sésame étant de 10% à 20% en poids.

2. Composition selon la revendication 1, caractérisée en ce que la proportion de vitamine E est d'environ 0,5% à environ 5% en poids, et avantageusement d'environ 1% à environ 3%.

3. Composition selon la revendication 1 ou la revendication 2, comprenant la fraction insaponifiable d'huile de sésame en mélange avec une (ou plusieurs) fraction(s) insaponifiable(s) provenant de toute huile, notamment d'une huile végétale, susceptible de contenir de la vitamine E, notamment de l'huile de soja, et avantageusement de l'huile de germe de blé.

4. Composition selon l'une des revendications 1 à 3, caractérisée en ce qu'elle comprend la fraction insaponifiable d'huile de germe de blé en mélange avec la fraction insaponifiable d'huile de sésame.

5. Composition selon l'une des revendications 1 à 4, caractérisée en ce qu'elles comprennent un mélange d'un concentrat d'huile de germe de blé et d'un concentrat d'huile de sésame, ces concentrats contenant respectivement les fractions insaponifiables d'huile de germe de blé et d'huile de sésame, ces concentrats étant tels qu'obtenus par distillation moléculaire de ces huiles, réalisée notamment à une température comprise entre environ 190°C et environ 260°C, avantageusement entre environ 200°C et environ 220°C, et sous vide de l'ordre d'environ 0,1 Pa à environ 1,5 Pa, avantageusement d'environ 0,13 Pa à environ 0,5 Pa.

6. Composition selon l'une des revendications 1 à 5, caractérisée en ce que les quantités en poids des fractions insaponifiables dans les concentrats d'huile de germe de blé et d'huile de sésame sont respectivement d'environ 15% à environ 25%, et d'environ 10% à environ 20%.

7. Composition selon l'une des revendications 1 à 6, caractérisée en ce que les quantités en poids des concentrats d'huile de germe de blé et d'huile de sésame varient respectivement entre environ 10% à environ 90%, et entre environ 90% à environ 10%, de manière à ce que le total des quantités de ces deux huiles soit de 100%.

8. Composition selon l'une des revendications 1 à 7, caractérisée en ce que les quantités en poids des concentrats d'huile de germe de blé et d'huile de sésame sont respectivement d'environ 50%.

9. Utilisation d'une composition comprenant la fraction insaponifiable d'huile de sésame en mélange avec de la vitamine E selon l'une des revendications 1 à 8, pour la préparation de compositions cosmétiques.

10. Composition cosmétique contenant toute composition comprenant la fraction insaponifiable d'huile de sésame en mélange avec de la vitamine E selon l'une des revendications 1 à 8, de préférence en association avec un véhicule physiologiquement acceptable.

11. Composition cosmétique selon la revendication 10, caractérisée en ce qu'elle est formulée pour l'usage topique, notamment sous forme de crèmes, d'émulsions, de pommades, ou encore sous une forme appropriée pour l'application labiale, et est utilisable notamment en tant que produits solaires pour la prévention contre les effets des UVA sur la peau, ainsi qu'en tant que produits solaires traitants, crèmes restructurantes, nutritives, antirides, et protectrices de jour.

12. Méthode de traitement esthétique caractérisée en ce qu'elle comprend l'application d'une composition selon la revendication 10 ou la revendication 11, sur la surface cutanée d'un individu.

13. Utilisation d'une composition comprenant la fraction insaponifiable d'huile de sésame en mélange avec de la vitamine E selon l'une des revendications 1 à 8, pour la préparation de compositions pharmaceutiques destinées à la prévention ou au traitement contre les effets des UVA sur la peau.

14. Utilisation d'une composition comprenant la fraction insaponifiable d'huile de sésame en mélange avec de la vitamine E selon l'une des revendications 1 à 8, pour la préparation de compositions pharmaceutiques destinées à la prévention du vieillissement de la peau.

15. Composition pharmaceutique caractérisée en ce qu'elle contient toute composition comprenant la fraction insaponifiable d'huile de sésame en mélange avec de la vitamine E selon l'une des revendications 1 à 8, de préférence en association avec un véhicule physiologiquement acceptable.

16. Utilisation d'une composition comprenant la fraction insaponifiable d'huile de sésame en mélange avec de la vitamine E selon l'une des revendications 1 à 8, pour la préparation de compositions alimentaires ou compléments nutritionnels.

17. Complément nutritionnel caractérisé en ce qu'il comprend toute composition comprenant la fraction insaponifiable d'huile de sésame en mélange avec de la vitamine E selon l'une des revendications 1 à 8.

## Claims

1. Composition comprising the unsaponifiable fraction of sesame oil mixed with vitamin E, the proportion of this unsaponifiable fraction of sesame oil being from 10% to 20% by weight.

2. Composition according to claim 1, characterized in that the proportion of vitamin E is approximately 0.5% to approximately 5% by weight, and advantageously approximately 1% to approximately 3%.

3. Composition according to claim 1 or claim 2, comprising the unsaponifiable fraction of sesame oil mixed with one (or more) unsaponifiable fraction(s) originating from any oil, in particular a vegetable oil, capable of containing vitamin E, in particular soya bean oil, and advantageously wheatgerm oil.

4. Composition according to one of claims 1 to 3 characterized in that it comprises the unsaponifiable fraction of wheatgerm oil mixed with the unsaponifiable fraction of sesame oil.

5. Composition according to one of claims 1 to 4, characterized in that they comprise a mixture of a wheatgerm oil and a concentrate of sesame oil, these concentrates respectively containing the unsaponifiable fractions of wheatgerm oil and sesame oil, these concentrates being as obtained by molecular distillation of these oils, carried out in particular at a temperature comprised between approximately 190°C and approximately 260°C, advantageously between approximately 200°C and approximately 220°C, and under a vacuum of the order of approximately 0.1 Pa to approximately 1.5 Pa, advantageously from approximately 0.13 Pa to approximately 0.5 Pa.

6. Composition according to one of claims 1 to 5, characterized in that the quantities by weight of the unsaponifiable fractions in the concentrates of wheatgerm oil and sesame oil are respectively approximately 15% to approximately 25%, and from approximately 10% to approximately 20%.

7. Composition according to one of claims 1 to 6, characterized in that the quantities by weight of the wheatgerm oil and sesame oil concentrates vary respectively between approximately 10% and approximately 90%, and between approximately 90% and approximately 10%, such that the total quantity of the two oils amounts to 100%.

8. Composition according to one of claims 1 to 7, characterized in that the quantities by weight of the wheatgerm oil and sesame oil concentrates are respectively approximately 50%.

9. Use of a composition comprising the unsaponifiable fraction of sesame oil mixed with vitamin E according to one of claims 1 to 8, for the preparation of cosmetic compositions.

10. Cosmetic composition containing any composition comprising the unsaponifiable fraction of sesame oil mixed with vitamin E according to one of claims 1 to 8, preferably in combination with a physiologically acceptable vehicle.

11. Cosmetic composition according to claim 10, characterized in that it is formulated for topical use, in particular in the form of creams, emulsions, ointments, or also in an appropriate form for application to the lips, and can be used in particular as sun products for the prevention of the effects of UVA's on the skin, and also as suncare products, restructuring, nutritive, anti-wrinkle and day protection creams.

12. Beauty treatment method characterized in that it comprises the application of a composition according to claim 10 or claim 11, on an individual's skin.

13. Use of a composition comprising the unsaponifiable fraction of sesame oil mixed with vitamin E according to one of claims 1 to 8, for the preparation of pharmaceutical compositions intended for the prevention or the treatment of the effects of UVA's on the skin.

14. Use of a composition comprising the unsaponifiable fraction of sesame oil mixed with vitamin E according to one of claims 1 to 8, for the preparation of pharmaceutical compositions intended for the prevention of ageing of the skin.

15. Pharmaceutical composition characterized in that it contains any composition comprising the unsaponifiable fraction of sesame oil mixed with vitamin E according to one of claims 1 to 8, preferably in combination with a physiologically acceptable vehicle.

16. Use of a composition comprising the unsaponifiable fraction of sesame oil mixed with vitamin E according to one of claims 1 to 8, for the preparation of food compositions and nutritional additives.

17. Nutritional additive characterized in that it comprises any composition comprising the unsaponifiable fraction of sesame oil mixed with vitamin E according to one of claims 1 to 8.

**Patentansprüche**

1. Zusammensetzung umfassend die nicht-verseifbare Fraktion von Sesamöl in Mischung mit Vitamin E, wobei der Anteil dieser nicht-verseifbaren Fraktion von Sesamöl zwischen 10 und 20 Gewichtsprozent liegt.

2. Zusammensetzung nach Anspruch 1, dadurch gekennzeichnet, daß der Anteil an Vitamin E von etwa 0,5 bis etwa 5 Gewichtsprozent und vorteilhafterweise von etwa 1 bis etwa 3 Gewichtsprozent beträgt.

3. Zusammensetzung nach Anspruch 1 oder 2, die die nicht-verseifbare Fraktion von Sesamöl in Mischung mit einer (oder mehreren) nicht-verseifbaren Fraktion(en) umfaßt, welche aus dem Gesamtöl stammt/stammen, insbesondere aus einem pflanzlichen Öl, das Vitamin E enthalten kann, insbesondere aus Sojaöl und vorteilhafterweise aus Weizenkeimöl.

4. Zusammensetzung nach einem der Ansprüche 1 bis 3, dadurch gekennzeichnet, daß sie die nicht-verseifbare Fraktion von Weizenkeimöl in Mischung mit der nicht-verseifbaren Fraktion von Sesamöl umfaßt.

5. Zusammensetzung nach einem der Ansprüche 1 bis 4, dadurch gekennzeichnet, daß sie eine Mischung eines Weizenkeimöl-Konzentrats und eines Sesamöl-Konzentrats umfaßt, wobei diese Konzentrate jeweils die nicht-verseifbaren Fraktionen von Weizenkeimöl und Sesamöl enthalten und durch Molekulardestillation dieser Öle erhältlich sind, die insbesondere bei einer Temperatur zwischen etwa 190 °C und etwa 260 °C, vorteilhafterweise zwischen etwa 200 °C und etwa 220 °C bei einem Vakuum in der Größenordnung von etwa 0,1 Pa bis etwa 1,5 Pa, vorteilhafterweise etwa 0,13 Pa bis etwa 0,5 Pa durchgeführt wird.

6. Zusammensetzung nach einem der Ansprüche 1 bis 5, dadurch gekennzeichnet, daß die Menge der nicht-verseifbaren Fraktionen in den Konzentraten des Weizenkeimöls und des Sesamöls jeweils etwa 15 bis etwa 25 Gewichtsprozent und etwa 10 bis etwa 20 Gewichtsprozent betragen.

7. Zusammensetzung nach einem der Ansprüche 1 bis 6, dadurch gekennzeichnet, daß die Mengen der Konzentrate des Weizenkeimöls und des Sesamöls jeweils zwischen etwa 10 und etwa 90 Gewichtsprozent und zwischen etwa 90 und etwa 10 Gewichtsprozent variieren, so daß die Gesamtmenge dieser zwei Öle 100 Gewichtsprozent betragen.

8. Zusammensetzung nach einem der Ansprüche 1 bis 7, dadurch gekennzeichnet, daß die Mengen der Konzentrate des Weizenkeimöls und des Sesamöls jeweils etwa 50 Gewichtsprozent betragen.

9. Verwendung einer Zusammensetzung umfassend die nicht-verseifbare Fraktion von Sesamöl in Mischung mit Vitamin E gemäß einem der Ansprüche 1 bis 8 für die Herstellung von kosmetischen Zusammensetzungen.

10. Kosmetische Zusammensetzung, welche die Gesamtzusammensetzung umfassend die nicht-verseifbare Fraktion von Sesamöl in Mischung mit Vitamin E gemäß einem der Ansprüche 1 bis 8, vorzugsweise zusammen mit einem physiologisch annehmbaren Medium enthält.

11. Kosmetische Zusammensetzung nach Anspruch 10, dadurch gekennzeichnet, daß sie für die topische Anwendung formuliert ist, insbesondere in Form von Cremes, Emulsionen, Pomaden oder auch in einer zur Anwendung auf die Lippen geeigneten Form, wobei die Zusammensetzung insbesondere als Sonnenschutzprodukt zum Schutz vor den Wirkungen von UVA-Strahlung auf der Haut sowie als Behandlungsprodukt nach Besonnung, Wiederaufbau-Creme, Nährcreme, Anti-Faltencreme und Tagesschutzcreme verwendbar ist.

12. Verfahren zur Schönheitsbehandlung, dadurch gekennzeichnet, daß es die Anwendung einer Zusammensetzung nach Anspruch 10 oder 11 auf die Hautoberfläche eines Individuums umfaßt.

13. Verwendung einer Zusammensetzung umfassend die nicht-verseifbare Fraktion von Sesamöl in Mischung mit Vitamin E gemäß einem der Ansprüche 1 bis 8 für die Herstellung von pharmazeutischen Zusammensetzungen zur vorbeugenden oder behandelnden Anwendung gegen die Wirkungen von UVA-Strahlung auf der Haut.

**14.** Verwendung einer Zusammensetzung umfassend die nicht-verseifbare Fraktion von Sesamöl in Mischung mit Vitamin E gemäß einem der Ansprüche 1 bis 8 für die Herstellung von pharmazeutischen Zusammensetzungen zur Vorbeugung gegen Hautalterung.

**15.** Pharmazeutische Zusammensetzung, dadurch gekennzeichnet, daß sie die Gesamtzusammensetzung umfassend die nicht-verseifbare Fraktion von Sesamöl in Mischung mit Vitamin E gemäß einem der Ansprüche 1 bis 8, vorzugsweise zusammen mit einem physiologisch annehmbaren Medium enthält.

**16.** Verwendung einer Zusammensetzung umfassend die nicht-verseifbare Fraktion von Sesamöl in Mischung mit Vitamin E gemäß einem der Ansprüche 1 bis 8 zur Herstellung von Nahrungsmittelzusammensetzungen oder Nährstoffergänzungsmitteln.

**17.** Nährstoffergänzungsmittel, dadurch gekennzeichnet, daß es die Gesamtzusammensetzung umfassend die nicht-verseifbare Fraktion von Sesamöl in Mischung mit Vitamin E gemäß einem der Ansprüche 1 bis 8 umfaßt.